# EUROPEAN PATENT APPLICATION

(11) **EP 1 782 797 A1**
(43) Date of publication of application: **09.05.2007**
(21) Application number: 05110254.9
(22) Date of filing: 02.11.2005
(51) Int. Cl.: A61K 9/14

(54) **Process for the preparation of sterile powdered pharmaceutical compounds.**

(71) Applicant: Pharmatex Italia Srl, 20121 Milano (IT)
(72) Inventor: De Tommaso, Vincenzo, 20052, Monza (IT)
(74) Representative: Serravalle, Marco

(57) **Abstract**

The invention relates to a process for the preparation of pharmaceutical powders, the process comprising: a) solubilization of the pharmaceutical compound in water or in an organic solvent at a concentration close to saturation; b) evaporating the solvent at constant temperature while subjecting the solution to ultrasound.

In another embodiment, the invention relates to a process for the preparation of sterile pharmaceutical compounds, the process comprising: a) solubilization of the pharmaceutical compound in water or in an organic solvent at a concentration close to saturation; b) sterilizing filtration of the solution by passing it through a hydrophobic filter (0,22 microns membrane); c) evaporating the solvent at constant temperature while subjecting the solution to ultrasound.

## Description

The present invention concerns a process for the preparation of powdered pharmaceutical compounds characterized by homogeneous and well defined particle size, which process makes use of ultrasounds in combination with evaporation of the solvent at constant temperature. More particularly, the process of the invention allows the preparation of sterile pharmaceutical powders.

The use of ultrasounds in the preparation of powders characterized by homogeneous particle size is well known in the art.

WO 03/101578 discloses a process for the production of crystalline material, the method comprising forming a saturated solution of the material, changing the temperature of the solution so it becomes supersaturated, and subjecting the solution to irradiation by high intensity ultrasound, the frequency of the ultrasound being scanned over a range of frequency.

WO 03/092581 discloses a process for the production of small crystals by mixing a solution of the desired substance with an anti-solvent in a fluidic vortex mixer. The liquid in the fluidic vortex mixer is subjected to high intensity ultrasound.

Both these processes, however, are not suitable for the direct preparation of sterile powders.

If sterilization is to be performed as a separate step in the preparation of the pharmaceutical compound, it is normally performed either by the use of gamma rays or by the use of ethylene oxide.

The use of gamma rays presents the important side effect of degrading part of the product. Furthermore it is an expensive and complex method. Ethylene oxide, on the other hand, requires high temperature and humidity. Also these conditions can cause degradation of the product.

It is therefore desirable to find a new process which results in a sterile pharmaceutical powder of defined particle size, at low cost and without degradation of the product.

The present invention provides a process for the preparation of small crystals of homogeneous particle size wherein a saturated liquid is subjected to evaporation of the solvent under vacuum while subjected to ultrasound. The supersaturated solution crystallizes by formation of crystals of homogeneous particle size. If the solution is subjected to sterilizing filtration through a 0.22 micron filter, it is possible to obtain a sterile powder of defined particle size.

The process of the invention is particularly advantageous over the prior art since at the end of the evaporation, all solid is recovered, resulting in a yield of 100% of the starting pharmaceutical substance. Furthermore, the process does not cause any degradation of the product.

Thus, in another embodiment of the invention, it is provided a process for the preparation of sterile pharmaceutical compounds, the process comprising: a) solubilization of the pharmaceutical compound in water or in an organic solvent at a concentration close to saturation; b) sterilizing filtration of the solution by passing it through a filter (0,22 microns membrane); c) evaporating the solvent at constant temperature while subjecting the solution to high intensity ultrasound.

Step a) can be performed at room temperature, at high temperature or even below room temperature. It is important that the room is classified as class C in accordance with European GMP.

The filtration is preferably performed by using a nitrogen pressure of from 0.5 to 1.5 bar. The filtrate is collected in a sterile vessel (class A room).

In step c) it is important that the temperature is such that the product is stable and the solvent is volatile. In certain cases, it is possible to apply vacuum to maintain the temperature low.

After precipitation, the product is dried until the solvent is completely removed.

In another embodiment of the invention, after sterilizing filtration a non-solvent is added to the solution. The addition of the non-solvent is not meant to cause precipitation, but to obtain, after evaporation of the solvent, a suspension of the pharmaceutical compound, which suspension can be further sonicated to obtain a reduction of the particle size of the compound.

Sonication of the solution can be performed either by placing the source of ultrasounds outside the crystallization vessel, or by introducing one or more probes into the vessel. Of course, when placing the ultrasounds probe outside the vessel, the efficiency is lower because of some dispersion of ultrasounds outside the vessel.

The powder is preferably passed through a sieve with from 70 to 250 micron meshes. In this way, possible agglomerates are removed.

### Examples

Solutions were sonicated by using a commercial probe Labsonic U B. Brown, power 0.52 W, repeat time 0.8".

The particle size of the powdered compounds was measured by using the following Coulter Counters: Beckman and Sympatec Helos.

### Example 1

In a 250 ml one neck round bottomed flask with a thermostatic jacket and a magnetic stirrer containing 140 ml of acetone, 2.5 g of triamcinolone acetonide were added. The suspension was heated to 50-60 °C under stirring until the solid was fully solubilized. The solution was then passed through a 0.22 micron filter and collected. The solution was kept at 25-30 °C under vacuum and acetone was evaporated, while the solution was subjected to ultrasound. After complete drying of acetone, the solid was passed through a 70 micron sieve.

### Example 2

In a 250 ml one neck round bottomed flask with a thermostatic jacket and a magnetic stirrer containing 140 ml of acetone, 2.5 g of Triamcinolone acetonide were added, the suspension heated to 50-60 °C until the solid was fully solubilized. The solution was then passed through a 0.22 micron filter and collected. 40 ml of sterile water were added under the action of ultrasound. The temperature of the solution was kept at 25 °C under vacuum and, always while subjecting the solution to ultrasound, acetone was evaporated. When acetone was fully evaporated, the suspension of triamcinolone acetonide in water was kept under the action of ultrasound for 1 h. Afterwards, the suspension was filtered through a membrane of 0.45 micron porosity. The solid product which remains on the filter was dried in oven under vacuum, and the powder was then passed through a 70 micron sieve.

### Example 3

In a 250 ml one neck round bottomed flask with a thermostatic jacket and a magnetic stirrer containing 35 ml tetrahydrofurane and 30 ml of acetone, 2.5 g of triamcinolone acetonide were added. The suspension was heated to 40-45 °C under stirring until the solid was fully solubilized. The solution was left to cool at room temperature and then passed through a 0.22 micron filter and collected. The solution was kept at 35-40 °C under vacuum and the organic solvents evaporated, while the solution was subjected to ultrasound. After complete drying, the solid was passed through a 70 micron sieve.

Specifications for triamcinolone acetonide requires the following granulometry: 90% <40 micron, 60% < 20 micron, 20% < 10 micron.

**Granulometry**

| | Example 1 | Example 2 | Example 3 |
|---|---|---|---|
| 20% | <7 micron | < 1.8 micron | <6 micron |
| 50% | < 18 micron | <7 micron | < 18 micron |
| 60% | < 19 micron | <9 micron | < 19 micron |
| 90% | <35 micron | <19 micron | <35 micron |
| 99% | <51 micron | <41 micron | <48 micron |

## Claims

1. Process for the preparation of pharmaceutical powders, the process comprising: a) solubilization of the pharmaceutical compound in water or in an organic solvent at a concentration close to saturation; b) evaporating the solvent at constant temperature while subjecting the solution to ultrasound.

2. Process for the preparation of sterile pharmaceutical compounds, the process comprising: a) solubilization of the pharmaceutical compound in water or in an organic solvent at a concentration close to saturation; b) sterilizing filtration of the solution by passing it through a filter (0,22 microns membrane); c) evaporating the solvent at constant temperature while subjecting the solution to ultrasound.

3. Process according to claims 1-2 wherein the solution is subjected to ultrasound by introducing one or more ultrasound probes in the solution.

4. Process according to claims 1-2 wherein the solution is subjected to ultrasound by placing the source of ultrasounds outside the crystallization vessel.

5. Process according to claims 2-4 wherein after filtration a sterile non-solvent is added and the final powder after step c) remains suspended in the non solvent.

6. Process according to claim 5 wherein the suspension obtained after step c) is further sonicated to reduce particle size of the powder.
